# EUROPÄISCHE PATENTANMELDUNG

(11) **EP 4 079 722 A1**
(43) Veröffentlichungstag der Anmeldung: **26.10.2022**
(21) Anmeldenummer: 21169385.8
(22) Anmeldetag: 20.04.2021
(51) Int. Cl.: C07C 267/00, C08G 18/02

(54) **VERFAHREN ZUR HERSTELLUNG VON CARBODIIMIDEN**

(71) Anmelder: LANXESS Deutschland GmbH, 50569 Köln (DE)
(72) Erfinder: LAUFER, Wilhelm, 67158 Ellerstadt (DE)

(57) **Zusammenfassung**

Die Erfindung betrifft ein neues Verfahren zur Herstellung von Carbodiimiden umfassend die Schritte der
a) Carbodiimidisierung von Isocyanaten in Gegenwart eines Katalysators,
b) Abtrennung des Katalysators und/oder monomeren Isocyanats vom Carbodiimid durch Destillation oder Extraktion unter Erhalt eines monomeres Isocyanat enthaltenden Rohcarbodiimids,
c) Zugabe von einem oder mehreren Alkoholen und teilweise oder vollständige Umsetzung des Alkohols mit dem monomeren Isocyanat des Rohcarbodiimids.

## Beschreibung

Die Erfindung betrifft ein neues Verfahren zur Herstellung von Carbodiimiden mit reduziertem Restisocyanatmonomerengehalt.

Carbodiimide haben sich in vielen Anwendungen bewährt, z.B. als Hydrolyseschutzmittel für thermoplastische Kunststoffe, Polyole, Polyurethane, Triglyceride und Schmierstofföle etc.

Die Synthese der Carbodiimide erfolgt nach dem Stand der Technik üblicherweise ausgehend von Isocyanaten, die durch basische oder heterocyclische Katalyse unter CO₂-Abspaltung carbodiimidisiert werden. Dabei können mono- oder polyfunktionelle Isocyanate zu monomeren oder polymeren Carbodiimiden umgesetzt werden.

Üblicherweise werden als Katalysatoren Alkali- oder Erdalkaliverbindungen, sowie heterocyclische Verbindungen, die Phosphor enthalten verwendet. Entsprechende Katalysatoren sind beispielsweise in Angew. Chem. 1962, 74, 801-806 und Angew. Chem. 1981, 93, 855-866 beschrieben.

Die Entfernung der als Rohstoff eingesetzten Isocyanate nach der durchgeführten Carbodiimidisierung aus dem Produkt ist mit hohem Aufwand verbunden, vor allem bei der Herstellung von polymeren Carbodiimiden. Das Ziel, den RestisocyanatmonomerenGehalt deutlich unter 0,1 Gew.-% zu reduzieren, ohne die Produktqualität (insbesondere die Farbzahl) zu beeinträchtigen wird in der Regel nur durch mehrmalige Kristallisationen unter starken Ausbeutenverlusten erzielt. Zur Herstellung von Isocyanatgruppen und Urethangruppen enthaltenden Carbodiimiden mit geringem Restisocyanatmonomer-Gehalt, können alternativ, wie in der EP 1451239 beschrieben, durch mehrfache Abdestillation der Monomere über Kurzwegverdampfer erhalten werden, was jedoch zeit- und kostenintensiv ist.

Aufgabe der vorliegenden Erfindung war es daher, ein verbessertes Verfahren zur Herstellung von Carbodiimiden mit reduziertem Gehalt an Isocyanaten bereitzustellen, welches die obigen Nachteile des Stands der Technik weitestgehend vermeidet und hohe Ausbeuten mit ökonomischer Durchführbarkeit und hoher Produktqualität ermöglicht.

Überraschenderweise wurde nun gefunden, dass die vorgenannte Aufgabe erfüllt wird durch ein Verfahren zur Herstellung von Carbodiimiden der Formel (I)

R^{III}-(-N=C=N-R^{I})ₙ-N=C=N-R^{II} (I),

wobei
- n: für eine Zahl von 1 bis 500, bevorzugt 2 bis 50, ganz besonders bevorzugt 4 bis 20 steht,
- R^{I}: für C₁-C₂₄-Alkylene und/oder C₅-C₁₂-Cycloalkylene, C₁-C₁₂-Alkyl-substituierte oder C₁-C₂₄-Oxyalkyl-substituierte Cycloalkylene, C₁-C₁₂-Alkyl-substituierte Arylene, C₁-C₂₄-Oxyalkyl-substituierte Arylene, C₇-C₁₈-Alkylaryl-substituierte Arylene sowie gegebenenfalls für C₁-C₁₂-Alkyl-substituierte über C₁-C₈-Alkylengruppen verbrückte Arylene, die in Summe 8 bis 30 Kohlenstoffatome aufweisen, sowie Arylen steht und
- R^{II},R^{III}: gleich oder unabhängig voneinander für C₁-C₂₄-Alkyl- und/oder C₅-C₂₄-Cyclo- oder C₁-C₁₂-Alkyl-substituierte oder C₁-C₂₄-Oxyalkyl-substituierte Cycloalkyle, C₁-C₁₂-Alkyl-substituierte oder C₁-C₂₄-Oxyalkyl-substituierte Aryle, C₇-C₁₈-Alkylarylsubstituierte Aryle sowie gegebenenfalls für C₁-C₁₂-Alkyl-substituierte über C₁-C₈-Alkylengruppen verbrückte Aryle, die in Summe 8 bis 30 Kohlenstoffatome aufweisen, sowie Aryl stehen,
umfassend die nachfolgenden Schritte:
a) Carbodiimidisierung von Isocyanaten in Gegenwart eines Katalysators zu einem Carbodiimid und monomeres Isocyanat enthaltenden Reaktionsgemisch,
b) zumindest teilweise Abtrennung des Katalysators und/oder monomeren Isocyanats vom Carbodiimid und monomeres Isocyanat enthaltenden Reaktionsgemisch durch Destillation oder Extraktion unter Erhalt eines monomeres Isocyanat enthaltenden Rohcarbodiimids,
c) Zugabe von einem oder mehreren Alkoholen zum monomeres Isocyanat enthaltenden Rohcarbodiimid und teilweise oder vollständige Umsetzung des Alkohols mit dem monomeren Isocyanat des Rohcarbodiimids.

Als Isocyanate werden dabei in Schritt a) monomere Verbindungen der Formeln OCN-R^{I}-NCO, OCN-R^{II} und OCN-R^{III} eingesetzt.

In einer alternativen Ausführungsformen werden in Schritt a) Verbindungen der Formel OCN-R^{I}-NCO in bereits oligomerisierter oder polymerisierter Form, d.h. als Verbindungen der Formel OCN-R^{I}-(-N=C=N-R^{I}-)₍ₙ₋₁₎-NCO zusammen mit Verbindungen der Formeln OCN-R^{II} und OCN-R^{III} carbodiimidisiert.

In weiteren alternativen Ausführungsformen werden in Schritt a) die Verbindungen der Formel OCN-R^{I}-NCO in bereits oligomerisierter oder polymerisierter Form und bereits einseitig terminierter Form eingesetzt. In diesen Fällen d.h. als Verbindungen der Formel R^{III}-(-N=C=N-R^{I}-)ₙ-NCO mit Verbindungen der Formel OCN-R^{II} oder Verbindungen der Formel R^{II}-(-N=C=N-R^{I}-)ₙ-NCO mit Verbindungen der Formel OCN-R^{III} cabodiimidisiert.

Die gegebenenfalls C₁-C₁₂-Alkyl-substituierten über Alkylengruppen verbrückten C₆-C₁₀-Arylene, die in Summe 8 bis 30 Kohlenstoffatome aufweisen, besitzen die allgemeine Struktur -Alkylen-Arylen-Alkylen-, wobei die Alkylene linear oder verzweigt sein können und die Arylengruppe bis zu vier C₁-C₁₂-Alkylsubstituenten aufweisen kann, unter der Voraussetzung, dass die Gesamtzahl der Kohlenstoffatome nicht mehr als 30 beträgt.

Bevorzugt sind dabei über Alkylengruppen verbrückten C₆-C₁₀-Arylene, die an der Arylengruppe keine Alkylgruppen aufweisen und bei denen die beiden Alkylengruppen jeweils 1 bis 6 Kohlenstoffatome aufweisen.

In einer bevorzugten Ausführungsform steht R^{I} für C₁-C₁₂-alkyl-substituierte C₆-C₁₂-Arylene, vorzugsweise für C₁-C₄-alkyl-substituierte C₆-C₁₂-Arylene, besonders bevorzugt für ein- bis dreifach C₁-C₄-alkyl-substituierte C₆-Arylene, und ganz besonders bevorzugt Di- und/oder Triisopropylphenylen.

In einer bevorzugten Ausführungsform stehen R^{II} und R^{II} unabhängig voneinander für C₁-C₁₂-alkyl-substituierte C₆-C₁₂-Aryle, vorzugsweise für C₁-C₄-alkyl-substituierte C₆-C₁₂-Aryle, besonders bevorzugt für ein- bis dreifach C₁-C₄-alkyl-substituierte C₆-Aryle, und ganz besonders bevorzugt Di- und/oder Triisopropylphenyl.

In einer weiter bevorzugten Ausführungsform steht R^{I} für C₁-C₁₂-alkyl-substituierte C₆-C₁₂-Arylene und R^{II} und R^{II} unabhängig voneinander für C₁-C₁₂-alkyl-substituierte C₆-C₁₂-Aryle. In einer weiter bevorzugten Ausführungsform steht R^{I} für C₁-C₄-alkyl-substituierte C₆-C₁₂-Arylene und R^{II} und R^{II} unabhängig voneinander für C₁-C₄-alkyl-substituierte C₆-C₁₂-Aryle. In einer weiter bevorzugten Ausführungsform steht R^{I} für ein- bis dreifach C₁-C₄-alkyl-substituierte C₆-Arylene und R^{II} und R^{II} unabhängig voneinander für ein- bis dreifach C₁-C₄-alkyl-substituierte C₆-Aryle.

In einer weiter bevorzugten Ausführungsform steht R^{I} für Di- und/oder Triisopropylphenylen und R^{II} und R^{II} unabhängig voneinander für Di- und/oder Triisopropylphenyl.

In einer bevorzugten Ausführungsform entsprechen die Carbodiimide der Formel (II), 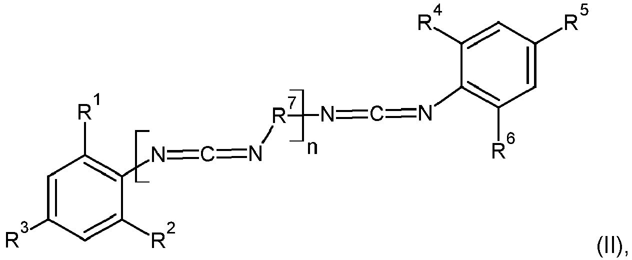 in der
- R¹, R², R³, R⁴, R⁵ und R⁶: gleich oder unabhängig voneinander H, C₁-C₂₀-Alkyl, C₃-C₂₀-Cycloalkyl, C₆-C₁₅-Aryl und/oder C₇-C₁₅-Aralkyl,
- R⁷: C₁-C₁₈-Alkylen, C₅-C₁₈-Cycloalkylen-, C₁-C₂₀-Alkyl-substituiertes Arylen und/oder C₇-C₁₈- Aralkylen, bevorzugt C₁-C₈-Alkyl-substituiertes Arylen und/oder C₇-C₁₈-Aralkylen, und
- n: eine Zahl im Bereich von 1 bis 500 bedeuten.

Vorzugsweise ist n eine Zahl im Bereich von 1 bis 50, bevorzugt von 3 - 20.

In den vorgenannten Ausführungsformen der Erfindung können auch Mischungen von Verbindungen der Formel (I) und/oder (II) mit unterschiedlichen Werten für n auftreten. In diesem Fall können sich bei der Ermittlung des Mittelwertes für n auch gebrochenen Zahlen ergeben.

Die Carbodiimidisierung von Isocyanaten in Gegenwart eines Katalysators in Schritt a) des erfindungsgemäßen Verfahrens erfolgt typischerweise in einer Kondensationsreaktion unter Abspaltung von CO₂, wie beispielsweise beschrieben in Angew. Chem. 93, S. 855 - 866 (1981) oder DE-A-11 30 594 oder Tetrahedron Letters 48 (2007), S. 6002 - 6004.

Die Carbodiimidisierung kann sowohl in Substanz als auch in einem Lösemittel durchgeführt werden. Ebenfalls möglich ist es, die Carbodiimidisierung zuerst in Substanz zu starten und während der Reaktion ein Lösemittel zuzusetzen. Geeignete Lösemittel können vom Fachmann leicht ermittelt werden. Als Beispiele für solche Lösemittel seien Petrolether, Benzol und/oder Alkylbenzole genannt.

Als Isocyanate werden besonders bevorzugt 1,3,5-Triisopropylphenyldiisocyanat (TRIDI), 2,6 Diisopropylphenylisocyanat (DIPI) oder 2,4,6 Triisopropylphenylisocyanat (TRIPI) verwendet.

Als Katalysatoren für die Carbodiimidisierung der Isocyanate zu Carbodiimiden der Formel (I) in Schritt a) sind in einer Ausführungsform der Erfindung starke Basen oder Phosphorverbindungen bevorzugt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Alkali-, Erdalkalioxide oder -Hydroxide, -Alkoholate oder -Phenolate, Carbonsäuremetallsalze und nicht basische Organometallverbindungen verwendet werden. Als Katalysator sind besonders Alkylphospholenoxide wie z. B. Methlyphospholenoxid bevorzugt.

Die Reaktion (Carbodiimidisierung) wird vorzugsweise in einem Temperaturbereich von 140°C bis 200°C, besonders bevorzugt von 160°C bis 180°C durchgeführt.

In einer weiteren Ausführungsform der Erfindung erfolgt zwischen Schritt a) und Schritt b) eine Filtration des Reaktionsprodukts aus Schritt a).

Die zumindest teilweise Abtrennung des Katalysators und/oder monomeren Isocyanats in Schritt b) bezweckt die Entfernung des Großteils dieser Stoffe. Unter Berücksichtigung der Verfahrenseffizienz wird dabei in Kauf genommen, dass keine vollständige Abtrennung des monomeren Isocyanats erfolgt. Die Abtrennung kann durch Destillation oder Extraktion erfolgen, wobei die Destillation bevorzugt ist. Bei der Destillation kann auch ggf. bei der Carbodiimidisierung verwendetes Lösungsmittel einfach abgetrennt werden. Die Destillation in Schritt b) erfolgt bevorzugt bei Temperaturen von 140°C bis 200 °C, besonders bevorzugt bei 160°C - 180°C. Sie wird in der Regel unter Vakuum bei einem Druck von 0,1 bis 50 mbar, bevorzugt 1 - 30 mbar, besonders bevorzugt 10 - 20 mbar durchgeführt. In einer bevorzugten Ausführungsform erfolgt die Destillation batchweise in einem Rührreaktor.

Die Zugabe und/oder Umsetzung des Alkohols in Schritt c) erfolgt bevorzugt bei Temperaturen, von 140°C bis 200°C, besonders bevorzugt von 160°C bis 180 °C. Besonders bevorzugt finden sowohl die Zugabe als auch die Umsetzung des Alkohols in Schritt c) bei Temperaturen im Bereich von 140°C bis 200°C, meist bevorzugt von 160°C bis 180°C statt.

Typischerweise werden in Schritt c) bezogen auf die Menge an enthaltenem Carbodiimid 0,1 - 5 Gew.-%, bevorzugt 0,2 - 2 Gew.-%, besonders bevorzugt 0,3 - 0,5 Gew.-% Alkohol(e) zugegeben.

Vorzugsweise werden als Alkohole aliphatische und/oder aromatische Alkohole, bevorzugt lineare, verzweigte und/oder cyclische aliphatische C₆-C₁₈ Alkohole, besonders bevorzugt n-Octanol, iso-Oktanol, Dodecanol, 2-Ethylhexanol, Oleylalkohol, Stearylalkohol und/oder Cyclohexanol eingesetzt.

In Anschluss an Schritt c) kann eine Destillation erfolgen, um einen ggf. vorhandenen Überschuss an Alkohol zu entfernen.

Die durch das erfindungsgemäße Verfahren erhaltenen Carbodiimide besitzen typischerweise einen Gehalt an monomerem Isocyanat von weniger als 1000 ppm, vorzugsweise weniger als 750 ppm, besonders bevorzugt weniger als 500 ppm, weiter bevorzugt weniger als 300 ppm und meist bevorzugt weniger als 100 ppm.

Die nachfolgenden Beispiele dienen der Erläuterung der Erfindung, ohne dabei limitierend zu wirken.

### Ausführungsbeispiele:

### Bestimmung des Restisocyanat Monomergehaltes

Der Restisocyanat Monomergehalt wurde mittels HPLC nach Umsetzung mit einer Reagenzlösung (1-Pyrdiyl-2-piperazin in 100 mL THF). Als Laufmittel wurde eine Mischung vom Amoniumacetatlösung und Methanol verwendet. Im Vorfeld wurde mit verschiedenen Konzentrationen des monomeren Isocyanates kalibriert.

**Beispiel-Carbodiimid:** Carbodiimid der Formel (II) mit R⁷ = Triisopropylphenylen, R¹, R², R³, R⁴, R⁵ und R⁶ = Isopropyl und n = ca. 18

### Beispiel 1: Carbodiimidisierung

Eine Mischung 1,3,5-Triisopropylphenyldiisocyanat (TRIDI) und 2,4,6 Triisopropylphenylisocyanat (TRIPI) wurde in Anwesenheit von ca. 0,2 % Methylphospholenoxid bei 160 °C carbodiimidisiert, bis ein NCO-Gehalt von < 1 % erreicht war. Das Reaktionsprodukt wurde 2 h bei 160 °C und 10 mbar destilliert

### Beispiel 2: Batch-Destillation

Das Carbodiimid aus Beispiel 1 wurde in einem Edelstahlkessel über einer kurzen Destillationsbrücke 5 h destilliert. Die Temperaturen in dem Destillationskessel lagen in einem Bereich von 160 °C bis 180 °C, der Enddruck lag bei ca. 10 mbar.

### Beispiel 3: Destillation über Dünnschichter / Kurzwegverdampfer

Das Carbodiimid aus Beispiel 1 wurde mittels einer Dünnschichter / Kurzwegverdampfer - Kombination bei ca. 180 °C und 1 mbar destilliert.

### Beispiel 4: Umkristallisation

Das Carbodiimid aus Beispiel 1 wurde in einem Edelstahlkessel in Toluol bei 60 °C gelöst und anschließend in Aceton bei 10 - 20 °C umkristallisiert. Nach der Fitration wurde das pulverförmige Carbodiimid im Trockner bei reduziertem Druck von 10 mbar mehrere Stunden getrocknet.

### Beispiel 5: Umsetzung mit Alkohol

Das Carbodiimid aus Beispiel 1 wurde in einem Edelstahlkessel mit 0,5 % 2-Ethylhexanol versetzt und ca. 30 min bei 160 °C gerührt

### Beispiel 6: Umsetzung mit Alkohol

Das Carbodiimid aus Beispiel 1 wurde in einem Edelstahlkessel mit 2,0 % 2-Ethylhexanol versetzt und ca. 30 min bei 160 °C gerührt

Der Restgehalt an monomerem Isocyanat wurde mittels HPLC bestimmt. Die Farbbestimmung wurde nach der CIE L*a*b* Methode ISO 11664-4 durchgeführt. Bewertet wurde der b-Wert.

Die Ergebnisse sind in Tabelle 1 nachstehend aufgeführt.

**Tabelle 1:**

| **Beispiel** | **Merkmale** | | |
|---|---|---|---|
| | Farbe, CIELab | Monomeres Isocyanat TRIPI in ppm, (Soll < 1000 ppm) | Ausbeute (%) |
| **1 (Vgl.)** | **6** | **ca. 1800** | **> 95** |
| **2 (Vgl.)** | **10** | **ca. 1300** | **> 95** |
| **3 (Vgl.)** | **8** | **< 100** | > 90 |
| **4 (Vgl.)** | **<4** | **< 100** | **< 60** |
| **5 (Erf.)** | **6** | **ca. 700** | **> 95** |
| **6 (Erf.)** | **6** | **< 100** | **> 95** |

| | | | |
|---|---|---|---|
| Vgl.: Vergleichsbeispiel, Erf.: Erfindungsgemäß | | | |

Wie aus Tabelle 1 ersichtlich, kann der Restisocyanat Monomergehalt auch nach sehr langen Destillationszeiten in dem einfachen Batch-Destillationsverfahren nicht erreicht werden. Darüber hinaus führen dies zu einer deutlichen Verschlechterung der Produktfarbe. Die gewünschten Qualitätsmerkmale werden erst mittles Umkristallisation oder mittels einer Dünnschichter/Kurzwegverdampfer-Kombination erreicht, diese Verfahren sind jedoch aufwändig und mit Ausbeuteverlusten verbunden. Die einfache Umsetzung mit geringen Mengen Alkohol (hier 2-Ethylhexanol) führt zu den gewünschten Ergebnissen unter Vermeidung der Nachteile der bekannten Verfahren.

## Patentansprüche

1. Verfahren zur Herstellung von Carbodiimiden der Formel (I)
R^{III}-(-N=C=N-R^{I}-)ₙ-N=C=N-R^{II} (I),
in der
n für eine Zahl von 1 bis 500, bevorzugt 2 bis 50, ganz besonders bevorzugt 4 bis 20 steht,
R^{I} für C₁-C₂₄-Alkylene und/oder C₅-C₁₂-Cycloalkylene, C₁-C₁₂-Alkyl-substituierte oder C₁-C₂₄-Oxyalkyl-substituierte Cycloalkylene, C₁-C₁₂-Alkyl-substituierte Arylene, C₁-C₂₄-Oxyalkyl-substituierte Arylene, C₇-C₁₈-Alkylaryl-substituierte Arylene sowie gegebenenfalls für C₁-C₁₂-Alkyl-substituierte über C₁-C₈-Alkylengruppen verbrückte Arylene, die in Summe 8 bis 30 Kohlenstoffatome aufweisen, sowie Arylen steht und
R^{II},R^{III} gleich oder unabhängig voneinander für C₁-C₂₄-Alkyl- und/oder C₅-C₂₄-Cyclo- oder C₁-C₁₂-Alkyl-substituierte oder C₁-C₂₄-Oxyalkyl-substituierte Cycloalkyle, C₁-C₁₂-Alkyl-substituierte oder C₁-C₂₄-Oxyalkyl-substituierte Aryle, C₇-C₁₈-Alkylaryl-substituierte Aryle sowie gegebenenfalls für C₁-C₁₂-Alkyl-substituierte über C₁-C₈-Alkylengruppen verbrückte Aryle, die in Summe 8 bis 30 Kohlenstoffatome aufweisen, sowie Aryl stehen,
umfassend die Schritte:
a) Carbodiimidisierung von Isocyanaten in Gegenwart eines Katalysators zu einem Carbodiimid und monomeres Isocyanat enthaltenden Reaktionsgemisch,
b) zumindest teilweise Abtrennung des Katalysators und/oder monomeren Isocyanats vom Carbodiimid und monomeres Isocyanat enthaltenden Reaktionsgemisch durch Destillation oder Extraktion unter Erhalt eines monomeres Isocyanat enthaltenden Rohcarbodiimids,
c) Zugabe von einem oder mehreren Akoholen zum monomeres Isocyanat enthaltenden Rohcarbodiimid und teilweise oder vollständige Umsetzung des Alkohols mit dem monomeren Isocyanat des Rohcarbodiimids.

2. Verfahren nach Anspruch 1, wobei in Schritt c) bezogen auf die Menge an enthaltenem Carbodiimid 0,1 - 5 Gew.-%, bevorzugt 0,2 - 2 Gew.-%, besonders bevorzugt 0,3 - 0,5 Gew.-% Alkohol(e) zugegeben werden.

3. Verfahren nach Anspruch 1 oder 2, wobei die Zugabe und/oder Umsetzung des Alkohols in Schritt c) bei Temperaturen im Bereich von 140 - 200 °C, besonders bevorzugt bei 160 - 180 °C erfolgt.

4. Verfahren nach einem oder mehreren der Ansprüche 1 bis 3, wobei als Alkohole aliphatische und/oder aromatische Alkohole, bevorzugt lineare, verzweigte und/ oder cyclische aliphatische C₆ - C₁₈ Alkohole, besonders bevorzugt n- Octanol, iso-Oktanol, Dodecanol, 2-Ethylhexanol, Oleylalkohol, Stearylalkohol und/oder Cyclohexanol eingesetzt werden.

5. Verfahren nach einem oder mehreren der Ansprüche 1 bis 4, wobei zwischen Schritt a) und Schritt b) eine Filtration des Reaktionsprodukts aus Schritt a) erfolgt.

6. Verfahren nach einem oder mehreren der Ansprüche 1 bis 5, wobei die Zugabe und/oder Umsetzung in Schritt c) unter Rühren erfolgt.

7. Verfahren nach einem oder mehreren der Ansprüche 1 bis 6, wobei die Carbodiimide der Formel (II), 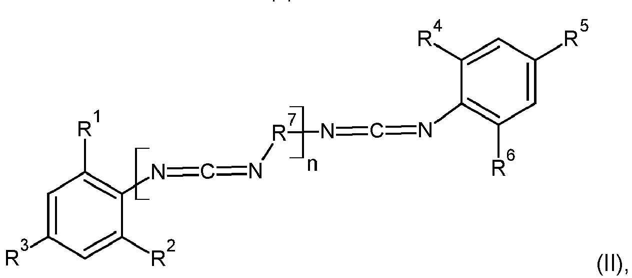 in der
R¹, R², R³, R⁴, R⁵ und R⁶ gleich oder unabhängig voneinander H, C₁-C₂₀-Alkyl, C₃-C₂₀-Cycloalkyl, C₆-C₁₅-Aryl und/oder C₇-C₁₅-Aralkyl ist,
R⁷ = C₁-C₁₈-Alkylen, C₅-C₁₈-Cycloalkylen-, C₁-C₂₀-Alkyl-substituiertes Arylen und/oder C₇-C₁₈-Aralkylen, bevorzugt C₁-C₈-Alkyl-substituiertes Arylen und/oder C₇-C₁₈- Aralkylen und n eine Zahl im Bereich von 1 bis 500 ist, entsprechen.

8. Verfahren nach Anspruch 7, wobei n eine Zahl im Bereich von 1 bis 50, bevorzugt von 3 - 20 ist.

9. Verfahren nach einem oder mehreren der Ansprüche 1 bis 8 wobei die Destillation in Schritt b) bei Temperaturen von 140 °C bis 200 °C, bevorzugt von 160 °C bis 180 °C erfolgt.

10. Verfahren nach einem oder mehreren der Ansprüche 1 bis 9, wobei die Destillation in Schritt b) bei einem Druck von 0,1 bis 50 mbar, bevorzugt von 1 bis 30 mbar, besonders bevorzugt von 10 bis 20 mbar durchgeführt wird.

11. Verfahren nach einem oder mehreren der Ansprüche 1 bis 10 wobei der Katalysator in Schritt a) ausgewählt ist aus der Gruppe bestehend aus Phospholenoxiden, Phospholidinen, Phospholinoxiden, sowie deren Sulfiden, tertiären Aminen, basisch reagierenden Metallverbindungen, Alkalioxiden, Alkalihydroxiden, Erdalkalioxiden, Erdalkalihydroxiden, Alkoholaten, Phenolaten, Carbonsäuremetallsalzen und nicht basischen Organometallverbindungen, vorzugsweise aus Alkylphospholenoxiden und besonders bevorzugt Methlyphospholenoxid.

12. Verfahren nach einem oder mehreren der Ansprüche 1 bis 11 wobei die Carbodiimidisierung in Gegenwart eines Lösungsmittels, vorzugsweise in Gegenwart von Benzinen, Benzol und/oder Alkylbenzol erfolgt.

13. Verfahren nach einem oder mehreren der Ansprüche 1 bis 12, wobei als Isocyanate 1,3,5-Triisopropylphenyldiisocyanat (TRIDI), 2,6 Diisopropylphenylisocyanat (DIPI) und/oder 2,4,6 Triisopropylphenylisocyanat (TRIPI) verwendet werden.

14. Verfahren nach einem oder mehreren der Ansprüche 1 bis 13, wobei im Anschluss am Schritt c) überschüssiger Alkohol durch Destillation entfernt wird.

15. Verfahren nach einem oder mehreren der Ansprüche 1 bis 14 wobei nach Schritt c) oder nach der ggf. im Anschluss an Schritt c) durchgeführten Destillation Carbodiimide erhalten werden, die einen Gehalt an monomerem Isocyanat von weniger als 1000 ppm, vorzugsweise weniger als 750 ppm, besonders bevorzugt weniger als 500 ppm, weiter bevorzugt weniger als 300 ppm und meist bevorzugt weniger als 100 ppm aufweisen.
